(19) Europäisches Patentamt
European Patent Office
Office européen des brevets

(11) Veröffentlichungsnummer: 0 379 132
A2

(12) **EUROPÄISCHE PATENTANMELDUNG**

(21) Anmeldenummer: 90100799.7

(51) Int. Cl.[5]: C07D 501/04, A61K 31/545

(22) Anmeldetag: 16.01.90

Patentansprüche für folgende Vertragsstaaten: ES + GR.

(30) Priorität: 19.01.89 DE 3901405

(43) Veröffentlichungstag der Anmeldung: 25.07.90 Patentblatt 90/30

(84) Benannte Vertragsstaaten: AT BE CH DE DK ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Adam, Friedhelm, Dr.
Rheingaustrasse 46
D-6238 Hofheim am Taunus(DE)
Erfinder: Blumbach, Jürgen, Dr.
Napean Sea Road, "Nilandri"
Bombay 400 006(IN)
Erfinder: Fischer, Gerd, Dr.
Johannesallee 6
D-6230 Frankfurt am Main 80(DE)
Erfinder: Dürckheimer, Walter, Dr.
Im Lerchenfeld 45
D-6234 Hattersheim am Main 80(DE)
Erfinder: Mencke, Burghard, Dr.
Hauptstrasse 2
D-5409 Holzappel(DE)
Erfinder: Isert, Dieter, Dr.
Hamburger Strasse 1
D-6236 Eschborn(DE)
Erfinder: Klesel, Norbert, Dr.
Bornstrasse 50
D-6238 Hofheim am Taunus(DE)
Erfinder: Seibert, Gerhard, Prof. Dr.
Gläserweg 21
D-6100 Darmstadt(DE)

(54) **Cephalosporinderivate und Verfahren zu ihrer Herstellung.**

(57) Cephalosporinderivate der allgemeinen Formel

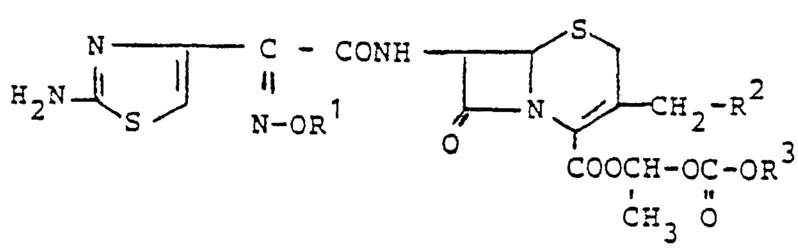

gegen bakterielle Infektionen wirksame pharmazeutische Präparate, die solche Cephemderivate enthalten, Verfahren zur Herstellung der Cephemderivate und der pharmazeutischen Präparate, sowie Verwendung der Gephemderivate zur Bekämpfung bakterieller Infektionen.

EP 0 379 132 A2

## Cephalosporinderivate und Verfahren zu ihrer Herstellung

Die Erfindung betrifft neue Cephalosporinderivate, die besonders für die orale Applikation geeignet sind, ein Verfahren zu ihrer Herstellung und pharmazeutische Zubereitungen, die solche Verbindungen enthalten.

Obwohl viele klinisch relevanten Cephalosporine mit breitem antibakteriellem Spektrum entwickelt worden sind, eignen sich die meisten von ihnen nur für eine parenterale Verabreichung, da sie nach oraler Gabe, wenn überhaupt, nur sehr unzureichend resorbiert werden. In vielen Fällen ist es jedoch wünschenswert, dem Patienten hochwirksame Antibiotika in oraler Form zu geben.

Die bislang bekannten Cephalosporin-Antibiotika erfüllen nicht alle Anforderungen, die an ein solches Medikament gestellt werden müssen, nämlich eine hohe antibakterielle Aktivität gegen grampositive (speziell Staphylokokken) und gramnegative Erreger und gleichzeitig eine gute Resorpotion im Magen-Darm-Trakt.

In einigen Fällen ist es gelungen, die Resorption eines Cephalosporins im Gastrointestinaltrakt durch Veresterung der 4-Carboxylgruppe zu erhöhen. Da die Cephalosporinester in der Regel per se keine antibiotische Wirksamkeit aufweisen, muß die Esterkomponente so gewählt werden, daß der Ester nach der Resorption durch körpereigene Enzyme, wie Esterasen, wieder rasch und vollständig zum Cephalosporin mit freier Carboxylgruppe zurückgespalten wird.

Das Ausmaß der enteralen Resorption von Cephalosporinen ist maßgeblich abhängig von der chemischen Struktur des Cephalosporins und der jeweiligen Esterkomponente. Schon kleine strukturelle Variationen am Cephalosporin-Grundgerüst oder in der Esterkomponente können die Resorption beeinflussen. Das Auffinden geeigneter Komponenten ist rein empirisch.

So führt beispielsweise die Einführung eines sauren Substituenten in die 7$\beta$-Seitenkette von Aminothiazolyl-Cephalosporinen, wie z.B. im Cefixime, zu einer enteral resorbierbaren Verbindung, während Verbindungen mit neutralen Seitenketten, wie z.B. im Cefuroxim, nur in Form von Prodrug-Estern enteral resorbiert werden. Die Dosis-Wirkungs-Proportionalität ist dabei nicht linear und die erzielten therapeutischen Serumspiegel sind nicht befriedigend. Carbonatester aus der Reihe der Aminothiazolyl-Cephalosporine werden beispielsweise in dem EP 134 420 erwähnt.

Wir fanden nun durch systematisch durchgeführte in vivo-Untersuchungen in verschiedenen Tierspecies eine enge Gruppe von oral applizierbaren Ceph-3-em-4-carbonsäureestern, die eine ausreichende chemische Stabilität besitzen und durch ausgewogene Lipid- und Wasserlöslichkeit rasch und in therapeutisch beachtlichem Maße im Magen-Darm-Trakt resorbiert werden.

Gegenstand der Erfindung sind demnach Cephemcarbonsäureester der allgemeinen Formel I

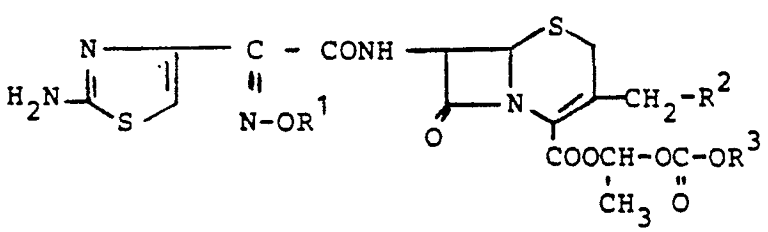

(I)

worin bedeuten

$R^1$ Wasserstoff oder Methyl und

$R^2$ Wasserstoff oder Methoxy, wobei immer einer der beiden Substituenten $R^1$ oder $R^2$ für Wasserstoff steht;

$R^3$ geradkettiges oder verzweigtes $C_1$-$C_5$-Alkyl, das substituiert sein kann durch $C_1$-$C_3$-Alkoxy, $C_3$-$C_8$-Cycloalkyl oder $C_2$-$C_7$-Cycloalkoxy; $C_3$-$C_8$-Cycloalkyl oder $C_2$-$C_7$-Cycloalkoxy,

wobei für den Fall, daß $R^1$ Wasserstoff und $R^2$ Methoxy ist, $R^3$ nicht $C_1$-$C_4$-Alkyl sein kann und worin die Gruppe -$OR^1$ in syn-Position steht, sowie deren physiologisch verträgliche Säureadditionssalze.

$R^3$ kann somit stehen

für $C_1$-$C_5$-Alkyl, das geradlinig oder verzweigt sein kann, wie z.B. Methyl, Ethyl, n-Propyl, i-Propyl, n-Butyl, 2-Butyl, 2-methyl-propyl, t-Butyl, n-Pentyl, 2-Pentyl, 2-Methylbutyl, 3-Methylbutyl, 1,1-Dimethylpropyl, 1,2-Dimethylpropyl oder 2,2-Dimethylpropyl, vorzugsweise für $C_1$-$C_4$-, insbesondere für $C_3$-$C_4$-Alkyl, wie z.B. n-Propyl, i-Propyl, n-Butyl, 2-Butyl, 2-Methyl-propyl, t-Butyl, wobei diejenigen Alkylreste noch eine Vorzugsstellung einnehmen, die in 1-Stellung durch Methyl substituiert sind und wobei die Alkylreste auch noch

substituiert sein können durch $C_1$-$C_3$-Alkoxy, wie z.B Methoxy, Ethoxy oder Propoxy, durch $C_3$-$C_8$-Cycloalkyl, wie z.B. Cyclopropyl, Cyclobutyl, Cyclopentyl, Cyclohexyl, Cycloheptyl oder Cyclooctyl, vorzugsweise durch $C_5$-$C_7$-Cycloalkyl, wie z.B. Cyclopentyl, Cyclohexyl oder Norbornyl, vorzugsweise Cyclopentyl oder Cyclohexyl, insbesondere Cyclohexyl, oder durch $C_2$-$C_7$-, vorzugsweise $C_4$-$C_5$-Cycloalkoxy, wie z.B. Tetrahydrofuranyl oder Tetrahydropyranyl, insbesondere Tetrahydropyranyl;
für $C_3$-$C_8$-Cycloalkyl, wie z.B. Cyclopropyl, Cyclobutyl, Cyclopentyl, Cyclohexyl, Cycloheptyl oder Cyclooctyl, vorzugsweise $C_5$-$C_7$-Cycloalkyl, wie z.B. Cyclopentyl, Cyclohexyl oder Norbornyl, vorzugsweise Cyclopentyl oder Cyclohexyl, insbesondere Cyclohexyl; oder
für $C_2$-$C_7$-, vorzugsweise $C_4$-$C_5$-Cycloalkoxy, wie z.B. Tetrahydrofuranyl oder Tetrahydropyranyl, insbesondere Tetrahydropyranyl.

Bevorzugt sind Verbindungen der allgemeinen Formel I, in der $R^1$ für Methyl und $R^2$ für Wasserstoff steht und $R^3$ die folgenden Bedeutungen besitzt:
$C_1$-$C_5$-, vorzugsweise $C_3$-$C_4$-Alkyl, das auch noch durch $C_1$-$C_3$-Alkoxy oder durch $C_5$-$C_7$-Cycloalkyl oder durch $C_4$-$C_5$-Cycloalkoxy substituiert sein kann;
$C_5$-$C_8$-, vorzugsweise $C_5$-$C_7$-Cycloalkyl oder $C_4$-$C_5$-Cycloalkoxy,
wobei diese für $R^3$ genannten Definitionen die vorstehend bei der Diskussion der Substituenten der allgemeinen Formel I angegebenen Bedeutungen besitzen.

Von besonderem Interesse sind unter den Verbindungen mit $R^1$ = Methyl und $R^2$ = Wasserstoff solche, bei denen $R^3$ für i-Propyl, 2-Butyl, 2-Methylpropyl, 1-Methoxy-2-propyl, Cyclopentyl oder Cyclohexyl steht.

Bevorzugt sind weiterhin Verbindungen der allgemeinen Formel I, in der $R^1$ für Wasserstoff, $R^2$ für Methoxy und $R^3$ für die Gruppe B

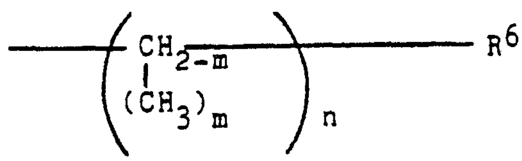

(B)

steht, worin bedeuten
n = 0 oder 1
m = 0 oder 1 und
$R^6$ = geradliniges oder verzweigtes $C_1$-$C_3$-Alkyl, das durch $C_1$-$C_3$-Alkoxy, $C_3$-$C_8$-Cycloalkyl oder $C_2$-$C_7$-Cycloalkoxy substituiert ist; $C_3$-$C_8$-Cycloalkyl oder $C_2$-$C_7$-Cycloalkoxy.

Für den Fall, daß n = 0 ist, kommen für $R^6$ folgende Bedeutungen in Betracht:
$C_3$-$C_8$-Cycloalkyl, vorzugsweise $C_5$-$C_7$-Cycloalkyl, wie z.B. Cyclopentyl, Cyclohexyl, Norbornyl, vorzugsweise Cyclopentyl oder Cyclohexyl, insbesondere Cyclohexyl;
$C_2$-$C_7$-Cycloalkoxy, vorzugsweise $C_4$-$C_5$-Cycloalkoxy wie z.B. Tetrahydrofuranyl oder Tetrahydropyranyl, insbesondere Tetrahydropyranyl.

Für n = 0 sind von besonderem Interesse solche Verbindungen, in denen in der Gruppe (B) $R^6$ für Cyclopentyl, Cyclohexyl, Tetrahydrofuranyl oder Tetrahydropyranyl, vorzugsweise Cyclohexyl oder Tetrahydropyranyl steht.

Für n = 0 kann $R^6$ auch stehen für geradliniges oder verzweigtes $C_1$-$C_3$-Alkyl, das durch $C_1$-$C_3$-Alkoxy, $C_3$-$C_8$-Cycloalkyl oder $C_2$-$C_7$-Cycloalkoxy substituiert ist, wobei die Alkoxy-, Cycloalkyl- und Cycloalkoxygruppen einschließlich ihrer auch hier in Betracht kommenden Vorzugsintervalle wie vorstehend für $R^3$ in der allgemeinen Formel I definiert sind.

Für $R^6$ sind bei n = 0 die folgenden substituierten Alkylgruppen von besonderem Interesse:
2-Methoxyethyl, Cyclopentylmethyl, Cyclohexylmethyl und Tetrahydropyranylmethyl.

Ist in der Gruppe (B) n = 1, so kommen für $R^6$ dieselben Bedeutungen wie für n = 0 in Betracht. Lediglich bei der substituierten $C_1$-$C_3$-Alkylgruppe ist bei n = 1 eine substituierte $C_1$-$C_2$-Alkylgruppe bevorzugt.

Für $R^6$ in der Bedeutung einer wie oben angegeben substituierten $C_1$-$C_3$-Alkylgruppe seien, insbesondere wenn n = 1 und m = 0 oder 1 ist, beispielhaft genannt:
Methoxy-methyl
2-Methoxy-ethyl
3-Methoxy-propyl

2-Methoxy-(2-methyl)-ethyl
2-Methoxy-(1-methyl)-ethyl
1-Methoxy-(1,1-dimethyl)-methyl
Ethoxy-methyl
2-Ethoxy-ethyl
3-Ethoxy-propyl
2-Ethoxy-(2-methyl)-ethyl
2-Ethoxy-(1-methyl)-ethyl
1-Ethoxy-(1,1-dimethyl)-methyl
(1-Propyloxy)-methyl
2-(1-Propyloxy)-ethyl
3-(1-Propyloxy)-propyl
2-(1-Propyloxy)-(2-methyl)-ethyl
2-(1-Propyloxy)-(1-methyl)-ethyl
1-(1-Propyloxy)-(1,1-dimethyl)-methyl
(2-Propyloxy)-methyl
2-(2-Propyloxy)-ethyl
3-(2-Propyloxy)-propyl
2-(2-Propyloxy)-(2-methyl)-ethyl
2-(2-Propyloxy)-(1-methyl)-ethyl
1-(2-propyloxy)-(1,1-dimethyl)-methyl
(Cyclopentyl)-methyl
2-(Cyclopentyl)-ethyl
3-(Cyclopentyl)-propyl
2-(Cyclopentyl)-(2-methyl)-ethyl
2-(Cyclopentyl)-(1-methyl)-ethyl
1-(Cyclopentyl)-(1,1-dimethyl)-methyl
(Cyclohexyl)-methyl
2-(Cyclohexyl)-ethyl
3-(Cyclohexyl)-propyl
2-(Cyclohexyl)-(2-methyl)-ethyl
2-(Cyclohexyl)-(1-methyl)-ethyl
1-(Cyclohexyl)-(1,1-dimethyl)-methyl
(4-Tetrahydropyranyl)-methyl
2-(4-Tetrahydropyranyl)-ethyl
3-(4-Tetrahydropyranyl)-propyl
2-(4-Tetrahydropyranyl)-(2-methyl)-ethyl
2-(4-Tetrahydropyranyl)-(1-methyl)-ethyl
1-(4-Tetrahydropyranyl)-(1,1-dimethyl)-methyl

vorzugsweise Methoxymethyl, 2-Methoxyethyl, Ethoxymethyl, 2-Ethoxyethyl, Cyclopentylmethyl, Cyclohexylmethyl und Tetrahydropyranylmethyl, wobei besonders bevorzugt sind Methoxymethyl, Cyclopentylmethyl, Cyclohexylmethyl oder Tetrahydropyranylmethyl.

Steht in der allgemeinen Formel I $R^1$ für Wasserstoff und $R^2$ für Methoxy, so sind demnach für $R^3$ ganz besonders bevorzugte Bedeutungen $C_5$-$C_7$-Cycloalkyl, insbesondere Cyclopentyl und Cyclohexyl, $C_4$-$C_5$-Cycloalkoxy, insbesondere Tetrahydropyranyl und durch $C_1$-$C_3$-Alkoxy substituiertes $C_1$-$C_3$-Alkyl, wie z.B. 2-Methoxy-(1-methyl)-ethyl.

Als physiologisch verträgliche Säureadditionssalze kommen die für Cephalosporin-Antibiotika bekannten Salze in Betracht, wie z.B. das Hydrochlorid, Sulfat, Maleinat, Citrat, Acetat oder Formiat. Ihre Herstellung erfolgt in an sich bekannter Weise durch Zusammengeben der entsprechenden Säure mit I in einem wäßrigen oder organischen Lösungsmittel oder einer geeigneten Lösungsmittelmischung.

Gegenstand der Erfindung ist weiterhin ein Verfahren zur Herstellung von Cephemcarbonsäureestern der allgemeinen Formel I

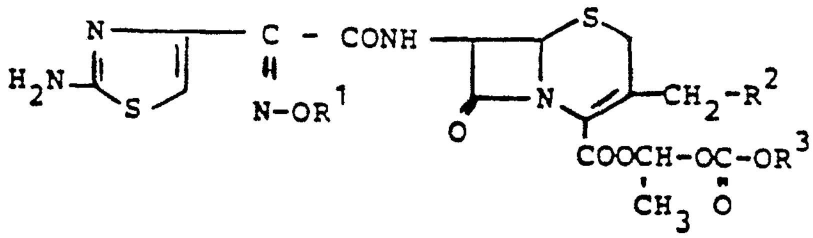

(I)

worin $R^1$ Wasserstoff oder Methyl und $R^2$ Wasserstoff oder Methoxy bedeuten, wobei immer einer der beiden Substituenten $R^1$ oder $R^2$ für Wasserstoff steht, $R^3$ für geradliniges oder verzweigtes $C_1$-$C_1$-Alkyl, das substituiert sein kann durch $C_1$-$C_3$-Alkoxy, $C_3$-$C_8$-Cycloalkyl oder $C_2$-$C_7$-Cycloalkoxy, für $C_3$-$C_8$-Cycloalkyl oder für $C_2$-$C_7$-Cycloalkoxy steht, wobei für den Fall, daß $R^1$ Wasserstoff und $R^2$ Methoxy ist, $R^3$ nicht $C_1$-$C_4$-Alkyl sein kann und worin die Gruppe -$OR^1$ in syn-Position steht, sowie von deren physiologisch verträglichen Säureadditionssalzen, das dadurch gekennzeichnet ist, daß man

a) eine Verbindung der Formel II

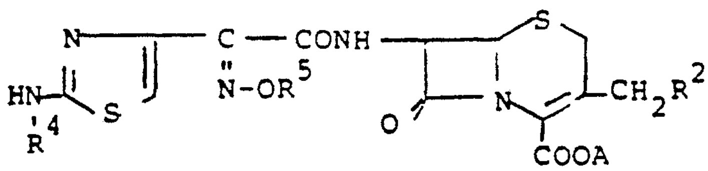

(II)

in der $R^2$ für Wasserstoff oder Methoxy, $R^4$ für Wasserstoff oder eine Aminoschutzgruppe, $R^5$ für Methyl oder eine leicht abspaltbare Gruppe und A für ein Kation steht, wobei $R^4$ nur dann für Wasserstoff stehen kann, wenn $R^5$ Methyl ist,
mit einer Verbindung der allgemeinen Formel III

$$X - \underset{\displaystyle CH_3}{\underset{|}{CH}} - OCO_2 - R^3 \qquad \text{(III)}$$

worin $R^3$ die obige Bedeutung besitzt und X für eine Abgangsgruppe steht, zu dem Ester der allgemeinen Formel IV umsetzt

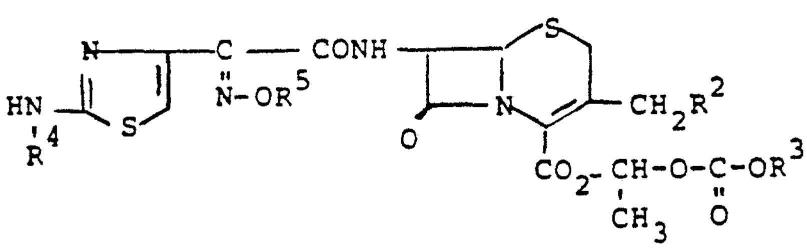

(IV)

und die Gruppen $R^4$ und $R^5$ in der Bedeutung einer Schutzgruppe oder leicht abspaltbaren Gruppe in an sich bekannter Weise entfernt oder

b) eine Verbindung der allgemeinen Formel V

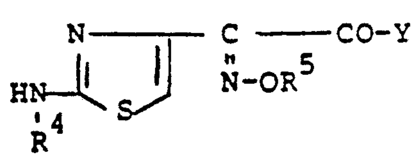

(V)

in der $R^4$ und $R^5$ die vorstehende Bedeutung besitzen und Y für eine aktivierende Gruppe steht, mit einer Verbindung der allgemeinen Formel VI

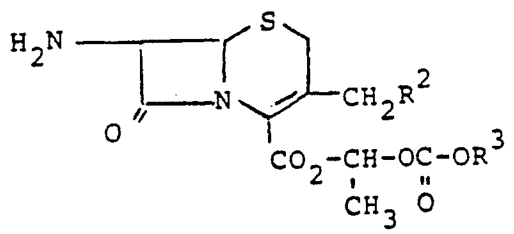

(VI)

in der $R^2$ und $R^3$ die vorstehende Bedeutung besitzen oder mit einem Salz dieser Verbindung, zu einer Verbindung der allgemeinen Formel IV umsetzt und die Gruppen $R^4$ und $R^5$ in der Bedeutung einer Schutzgruppe oder einer leicht abspaltbaren Gruppe in an sich bekannter Weise abspaltet, oder

c) eine Verbindung der allgemeinen Formel VII

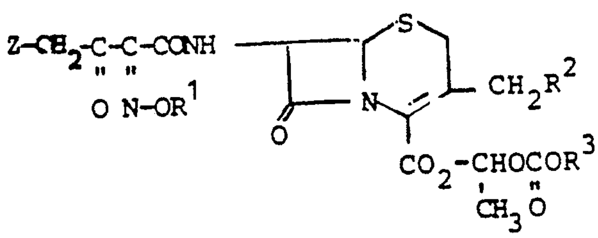

(VII)

in der Z für Halogen steht und $R^1$, $R^2$ und $R^3$ die vorstehende Bedeutung besitzen, mit Thioharnstoff zu Verbindungen der allgemeinen Formel I umsetzt und - falls erwünscht - die erhaltenen Verbindungen in ein physiologisch verträgliches Säureadditionssalz überführt.

In den allgemeinen Formeln II, IV und V steht $R^4$ für eine aus der Peptid- und Cephalosporinchemie bekannte Aminoschutzgruppe, vorzugsweise für Formyl, Chloracetyl, Bromacetyl, Trichloracetyl, Trifluoracetyl, Benzyloxycarbonyl, tert.-Butoxycarbonyl oder Trityl und $R^5$ für eine ebenfalls aus der Peptid- und Cephalosporinchemie bekannte leicht abspaltbare Gruppe, vorzugsweise für Benzhydryl, Trityl, Tetrahydropyranyl oder 1-Methoxy-1-methyl-ethyl. Besonders bevorzugt für $R^4$ ist Trityl und Chloracetyl, für $R^5$ Trityl und 1-Methoxy-1-methyl-ethyl.

In Formel III hat X die Bedeutung einer für Veresterungen allgemein bekannten Abgangsgruppe, wie beispielsweise Chlor, Brom, Jod, Phenylsulfonyloxy, p-Toluol-sulfonyloxy oder Methylsulfonyloxy, vorzugsweise Chlor, Brom oder Jod, insbesondere Jod.

Als Basen, die dem Kation A in der allgemeinen Formel II zugrundeliegen, seien z.B. genannt Natriumhydrogencarbonat, Kaliumhydrogencarbonat, Natriumcarbonat, Kaliumcarbonat, gegebenenfalls substituierte, alkylierte Aminbasen, wie z.B. Trimethylamin, Triethylamin, Diisopropylamin, Ethyldiisopropylamin, N,N-Dimethylanilin, N,N-Dimethylbenzylamin, 1,5-Diazabicyclo[4,3,0]non-5-en (DBN), 1,8-Diazabicyclo-[5,4,0]undec-7-en (DBU), Pyridin, Picolin oder 2,6-Dimethylpyridin. Bevorzugte Basen sind Natrium- oder Kaliumhydrogencarbonat, Natrium- oder Kaliumcarbonat, Triethylamin, N,N-Dimethylanilin, DBN oder DBU.

Durch Umsetzung der freien Carbonsäuren mit diesen Basen erhält man die Salze der allgemeinen Formel II, in der A für ein Kation, wie beispielsweise Natrium oder Kalium, aber auch für Magnesium oder Calcium oder für ein gegebenenfalls substituiertes alkyliertes Ammoniumion, wie beispielsweise Ammonium, Trimethylammonium, Triethylammonium, Tetrabutylammonium, Diisopropylammonium, Ethyldiisopropylammonium, Diazabicyclo[0,3,4]nonenium oder Diazabicyclo[0,4,5]undecenium steht. Bevorzugte Bedeutungen von A sind Natrium, Kalium, Triethylammonium, N,N-Dimethylanilinium, sowie das DBN- und DBU-ion.

In Verbindungen der Formel VII steht Z für ein Halogenatom, vorzugsweise für Brom oder Chlor.

Die Umsetzung der Verbindungen der Formel II mit den Verbindungen der Formel III kann in einem organischen Lösungsmittel bei etwa -20 bis etwa +50°C, bevorzugt bei etwa 0°C bis Raumtemperatur durchgeführt werden. Als Lösungsmittel können beispielsweise dienen Ketone, wie beispielsweise Aceton oder Methylethylketon, N,N-Dimethylformamid (DMF), N,N-Dimethylacetamid (DMA), N-Methylpyrrolidon oder Dimethylsulfoxid (DMSO). Bevorzugt sind DMF, DMA, N-Methylpyrrolidon und DMSO. Besonders

bevorzugt ist DMF.

Die Abspaltung der Gruppen $R^4$ und $R^5$ aus den erhaltenen Verbindungen der Formel IV erfolgt in an sich bekannter Weise nach bekannten Methoden der Peptid- und Cephalosporinchemie, z.B. mit Trifluoressigsäure, verdünnter Salzsäure, vorzugsweise mit Ameisensäure unter Zugabe von etwas Wasser.

Setzt man eine Verbindung der Formel V mit einer Verbindung der Formel VI um, so stellt Y eine die Carboxylgruppe aktivierende Gruppe dar, wie sie aus der Peptid- und Cephalosporinchemie für entsprechende Reaktionen bekannt ist, beispielsweise ein Halogenid, vorzugsweise Chlorid, eine aktivierende Estergruppe, beispielsweise mit 1-Hydroxybenzotriazol oder ein gemischtes Anhydrid, beispielsweise mit Benzolsulfonsäure oder Toluolsulfonsäure. Die Aktivierung der Carboxylgruppe ist auch in literaturbekannter Weise über die Zugabe eines Kondensationsmittels, wie z.B. eines Carbodiimids, möglich.

Die Verbindung der allgemeinen Formel VI läßt sich als solche oder in Form eines Salzes, beispielsweise des Tosylats, Hydrochlorids oder Hydrojodids einsetzen, wobei die Verwendung kristalliner Salze im Hinblick auf die Reinheit der Produkte vorteilhaft sein kann.

Die Umsetzung von Verbindungen der Formel V mit solchen der Formel VI kann in einem organischen Lösungsmittel, wie beispielsweise Methylenchlorid, Chloroform, Aceton, Methylethylketon, Dimethylformamid, Dimethylacetamid oder Wasser, oder auch in Mischungen dieser Lösungsmittel erfolgen.

Die Acylierungsreaktion kann zweckmäßigerweise bei Temperaturen von etwa -50 bis etwa +50 °C, vorzugsweise -40 bis +30 °C, gewünschtenfalls in Anwesenheit einer Base, wie beispielsweise Triethylamin oder Pyridin, durchgeführt werden. Der Basenzusatz dient dazu, die bei der Kondensation freiwerdende acide Komponente zu binden.

Der Ringschluß von Verbindungen der allgemeinen Formel VII mit Thioharnstoff kann nach an sich bekannten Verfahren, wie sie beispielsweise in der EP-PS 134 420 beschrieben sind, durchgeführt werden. Er gelingt z.B. glatt bei Temperaturen von etwa 0 bis 30 °C, vorzugsweise etwa 5 °C in organischen Lösungsmitteln, vorzugsweise aprotisch polaren Solventien, wie z.B. Dimethylformamid, Dimethylacetamid, Acetonitril oder Aceton.

Die Verbindungen der Formel III können in an sich bekannter Weise hergestellt werden, indem man z.B. Chlorameisensäure-1-chlorethylester

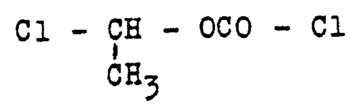

mit Alkoholen der allgemeinen Formel VIII

$R^3$ - OH (VIII)

in der $R^3$ die oben angegebene Bedeutung hat, umsetzt.

Die Reaktion wird zweckmäßigerweise in einem organischen Lösungsmittel wie einem halogenierten Kohlenwasserstoff, beispielsweis Methylenchlorid oder Chloroform, gegebenenfalls in Anwesenheit einer Base, beispielsweise Pyridin oder Triethylamin bei einer Temperatur von -20 bis +30 °C durchgeführt.

Verbindungen der Formel III können auch durch Halogenaustausch hergestellt werden. Beispielsweise kann eine Verbindung III, in der X für Jod oder Brom steht durch Umsetzen der entsprechenden Verbindung III, in der X für Chlor steht, mit einem Jodid- oder einem Bromidsalz, wie beispielsweise Natriumjodid oder Natriumbromid, gegebenenfalls in Gegenwart eines Katalysators, beispielsweise Zinkchlorid, hergestellt werden.

Die Herstellung der Ausgangsverbindungen der allgemeinen Formel II ist in der EP-PS 34 536 beschrieben.

Die Herstellung von Ausgangsverbindungen der allgemeinen Formel V mit der aktivierten Carboxylgruppe erfolgt in literaturbekannter Weise, die zu den Verbindungen der Formel VI führende Veresterung auf dieselbe Weise, wie sie für die Herstellung der Ester der allgemeinen Formel IV beschrieben wurde.

Die Verbindungen der allgemeinen Formel VII lassen sich nach an sich bekannten Verfahren herstellen. So kann man beispielsweise (vgl. EP-PS 134 420) Diketen mit Brom, und das erhaltene Zwischenprodukt darin mit einer Verbindung der allgemeinen Formel VI umsetzen, wobei man ein Vorprodukt der Formel

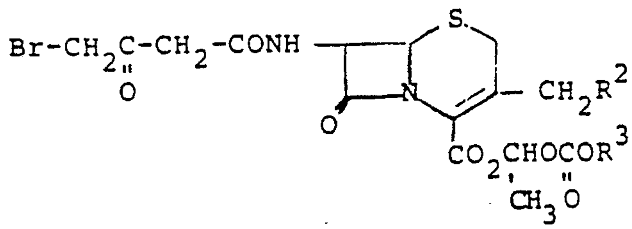

in der $R^2$ Wasserstoff oder Methoxy bedeutet, erhält, das anschließend durch Nitrosierung (vgl. ebenfalls EP-PS 134 420) in eine Verbindung der allgemeinen Formel VII überführt wird.

Die Ceph-3-em-4-carbonsäureester der allgemeinen Formel I besitzen eine Reihe physikochemischer und biologischer Eigenschaften, die sie zu wertvollen Cephalosporin-Antibiotika zur oralen Verabreichung machen. Sie sind stabile, farblose und in gängigen organischen Lösungsmitteln gut lösliche Verbindungen, die im Darm resorbiert, im Serum rasch zu antibiotisch aktiven Cephalosporin-Derivaten der Formel

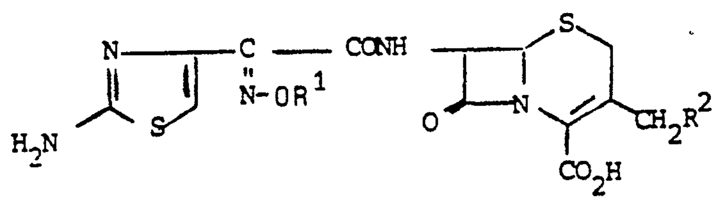

worin $R^1$ und $R^2$ die in Formel I angegebenenn Bedeutungen besitzen, gespalten werden und sich daher hervorragend zur Behandlung von bakteriellen Infektionskrankheiten, wie z.B. der Infektion der Atemwege oder des Urogenitaltraktes eignen.

Die erfindungsgemäßen Verbindungen werden oral verabreicht in Form von üblichen pharmazeutischen Zubereitungen, wie z.B. Kapseln, Tabletten, Pulvern, Sirupen oder Suspensionen. Die Dosis hängt ab vom Alter, den Symptomen und dem Körpergewicht des Patienten sowie von der Dauer der Behandlung. Sie liegt jedoch in der Regel zwischen etwa 0,2 g und etwa 5 g täglich, vorzugsweise zwischen etwa 0,5 g und etwa 3 g täglich. Die Verbindungen werden vorzugsweise in aufgeteilten Dosen verabreicht, beispielsweise 2 bis 4-mal täglich, wobei die Einzeldosis beispielsweise zwischen 50 und 500 mg Wirkstoff enthalten kann.

Die oralen Zubereitungen können die üblichen Trägerstoffe und/oder Verdünnungsmittel enthalten. So kommen beispielsweise für Kapseln oder Tabletten in Betracht Bindemittel, wie z.B. Gelatine, Sorbitol, Polyvinylpyrrolidon oder Carboxymethylcellulose, Verdünnungsmittel, wie z.B. Lactose, Zucker, Stärke, Calciumphosphate oder Polyethylenglykol, Gleitstoffe, wie z.B. Talkum oder Magnesiumstearat, für flüssige Zubereitungen z.B. wäßrige oder ölige Suspensionen, Sirupe oder ähnliche bekannte Zubereitungsformen.

Die folgenden Beispiele dienen zur weiteren Erläuterung der Erfindung, schränken sie jedoch nicht darauf ein.

A) Herstellung von Ausgangssubstanzen

**Herstellungsbeispiel 1**

1-Chlorethyl-(1-methoxy-2-propyl)-carbonat

7,7 ml (70 mmol) 1-Chlorethylchloroformat und 6,6 ml (70 mmol) 1-Methoxy-2-propanol wurden in 40 ml trockenem Methylenchlorid gelöst und auf 0 °C gekühlt. Dann tropfte man bei 0 - 5 °C eine Mischung von 5,8 ml (72 mmol) Pyridin und 20 ml trockenem Methylenchlorid zu, rührte noch 2 Stunden unter Eiskühlung und saugte das gebildete Pyridiniumhydrochlorid ab. Das Filtrat wurde zweimal gewaschen ($H_2O$) und im Wasserstrahlvakuum destilliert. Man erhielt 8,8 g (64 %) der Titelverbindung ($Kp_{30}$ = 100 - 105 °C).

$^1$H-NMR ($CDCl_3$): $\delta$ = (ppm)

6.46 (q, CH-Cl)

4.93 (m, $CH-OCO_2$)
3.63 (d, $CH_2-O$)
3.40 (s, $OCH_3$)
1.83 (d, $CH_3-CHCl$)
1.46 (d, $CH_3-CHOCO_2$).

Analog Herstellungsbeispiel 1 wurden die in der Tabelle 1 zusammengestellten Verbindungen hergestellt:

Tabelle 1

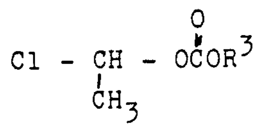

| Beisp. Nr. | $R^3$ | Ausbeute | NMR ($CDCl_3$) δ = (ppm) |
|---|---|---|---|
| 1 | 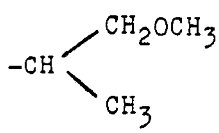 | 64 % | 6.46 (q, CH-Cl)<br>4.93 (m, $CH-OCO_2$)<br>3.63 (d, $CH_2-O$)<br>3.40 (s, $OCH_3$)<br>1.83 (d, $CH_3-CHCl$)<br>1.46 (d, $CH_3-CHOCO_2$). |
| 2 | 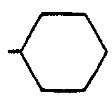 | 74 % | 6.48 (q, CH-Cl)<br>4.7 (m, $CH-OCO_2$)<br>2.2-1.0 (m, Cyclohexyl-H)<br>1.83 (d, $CH_3-CHCl$, verdeckt). |
| 3 | 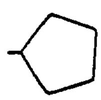 | 45 % | 6.83 (q, CH-Cl)<br>5.16 (m, $CH-OCO_2$)<br>2.0-1.5 (m, $CH_2$)<br>1.83 (d, $CH_3-CHCl$, verdeckt). |
| 4 | 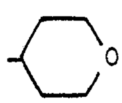 | 47 % | 6.43 (q, CH-Cl)<br>5.08-4.6 (m, $CH-OCO_2$)<br>4.16-3.3 (m, $(CH_2)_2O$)<br>2.16-1.4 (m, $C(CH_2)_2$)<br>1.83 (d, $CH_3-CHCl$, verdeckt). |
| 6 | 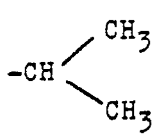 | 57 % | 6.49 (q, CH-Cl)<br>5.08-4.6 (m, $CH-OCO_2$)<br>2.16-1.4 (m, $C(CH_2)_2$)<br>1.80 (d, $CH_3-CHCl$, verdeckt).<br>1.33 (d, $CH(CH_3)_2$) |

Tabelle 1 (Fortsetzung)

| Beisp. Nr. | $R^3$ | Ausbeute | NMR ($CDCl_3$) δ = (ppm) |
|---|---|---|---|
| 7 | 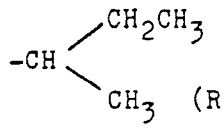 (R,S) | 60 % | 6.43 (q, CH-Cl)<br>4.78 (m, O-CH-)<br>1.83 (d, $CH_3$-CHCl, verdeckt).<br>1.5 - 1.8 (m, $CH_2$)<br>1.3 (2 x t, O-CH($CH_3$)<br>0.92 (2 x t, $CH_2$-$CH_3$). |
| 9 | $-CH_2CH_2CH_3$ | 62 % | 6.48 (q, CH-Cl)<br>4.16 (t, $CH_2$O)<br>1.83 (d, $CH_3$-CHCl, verdeckt).<br>1.67 (m, $CH_2$-$CH_2$-$CH_2$ verdeckt)<br>0.97 (t, $CH_3$) |
| 10 | $-CH_2CH_2OCH_3$ | 78 % | 6.48 (q, CH-Cl)<br>4.3 (m, $CH_2$-$OCH_3$)<br>3.6 (m, $CO_2CH_2$)<br>3.43 (s, $OCH_3$)<br>2.16-1.4 (m, C($CH_2$)$_2$)<br>1.81 (d, $CH_3$-CHCl, verdeckt). |
| 11 | 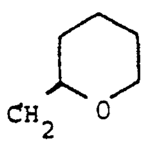 | 53 % | 6.48 (q, CH-Cl)<br>4.17 (s, $CH_2$)<br>3.5 - 4.1 (m, $CH_2OCH_2$)<br>1.5 - 2.1 (m, $CH_2CH_2$). |
| 12 | 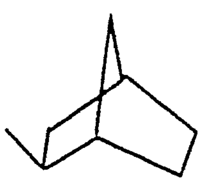 | 40 % | 6.48 (q, CH-Cl)<br>4.4 - 4.7 (m, CH-O)<br>3.5 - 3.9 (m, CH)<br>um 2.4 (m, CH)<br>1.81 (d, $CH_3$-CHCl, verdeckt).<br>1.0 - 1.9 (m, $CH_2$) |

Tabelle 1 (Fortsetzung)

| Beisp. Nr. | $R^3$ | Ausbeute | NMR ($CDCl_3$) δ = (ppm) |
|---|---|---|---|
| 13 | 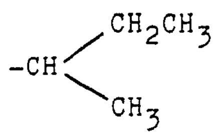 (R) | 62 % | 6.43 (q, CH-Cl)<br>4.78 (m, O-CH-)<br>1.83 (d, $CH_3$-CHCl, verdeckt).<br>1.5 - 1.8 (m, $CH_2$)<br>1.3 (2 x t, O-CH($CH_3$)<br>0.92 (2 x t, $CH_2$-$CH_3$). |
| 14 | 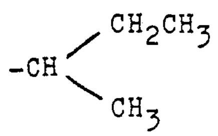 (S) | 58 % | 6.43 (q, CH-Cl)<br>4.78 (m, O-CH-)<br>1.83 (d, $CH_3$-CHCl, verdeckt).<br>1.5 - 1.8 (m, $CH_2$)<br>1.3 (2 x t, O-CH($CH_3$)<br>0.92 (2 x t, $CH_2$-$CH_3$). |
| 15 | $-CH_2CH_2CH_2OCH_2CH_2CH_3$ | 32 % | 6.48 (q, CH-Cl)<br>4.33 (t, $CH_2$)<br>3.53 (t, $CO_2CH_2$)<br>3.47 (q, $CH_2$)<br>1.87 (m, $CH_2$)<br>1.81 (d, $CH_3$-CHCl, verdeckt).<br>1.17 (t, $CH_3$) |
| 16 | 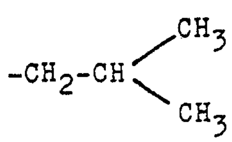 | 52 % | 6.48 (q, CH-Cl)<br>4.03 (d, $CH_2$)<br>3.6 (m, $CO_2CH_2$)<br>1.81 (d, $CH_3$-CHCl, verdeckt).<br>1.0 (q, $CH_3$) |

B) Ausführungsbeispiele

**1. Verbindungen der allgemeinen Formel IV**

**Beispiel 1**

7-<(2-(2-Tritylaminothiazol-4-yl)-2-(Z)-1-methyl-1-methoxyethoximinoacetamido)>-3-methoxymethyl-3-cephem-4-carbonsäure-1-(1-methoxyprop-2-yloxycarbonyloxy)ethylester

Schritt A

Zu einer Suspension von 560 mg (41 mmol) wasserfreiem Zinkchlorid in 33 ml Schwefelkohlenstoff gab man nacheinander 4,3 g (28.3 mmol) Natriumjodid und 4,3 g (22 mmol) 1-Chlorethyl-(1-methoxy-2-propyl)-carbonat. Man rührte 2 Stunden unter Stickstoffatmosphäre, dann goß man die Reaktionsmischung in ein Gemisch aus 300 ml 9 %iger $NaHCO_3$-Lösung und 300 ml Ether und trennte die Phasen. Die organiche Phase wurde gewaschen ($NaHCO_3$-, Natriumthiosulfat-und NaCl-Lösung) und getrocknet ($MgSO_4$). Das Lösungsmittel zog man im Vakuum bei 20° C ab und erhielt rohes 1-Jodethy-(1-methoxy-2-propyl)-carbonat als farbloses Öl, das ohne Reinigung sofort weiterverwendet wurde (Schritt B).

Schritt B

Das aus Schritt A erhaltene Rohprodukt wurde in 5 ml trockenem DMF aufgenommen und unter Eiskühlung zu einer Lösung von 3,8 g (5 mmol) Kalium-7-<(2-(2-tritylaminothiazol-4-yl)-2-(Z)-1-methyl-1-methoxy-ethoxyiminoacetamido)>-3-methoxymethyl-3-cephem-4-carboxylat in 15 ml trocknem DMF gegeben. Nach 10 Minuten rührte man das Reaktionsgemisch in eine Mischung von 200 ml 9 %iger $NaHCO_3$-Lösung und 100 ml Essigester ein. Die organische Phase wurde gewaschen ($NaHCO_3$- und NaCl-Lösung), getrocknet ($MgSO_4$) und das Lösungsmittel im Vakuum abgezogen. Das erhaltene Ol wurde unter Eiskühlung mit 100 ml Ether verrieben und zur Kristallisation gebracht. Den Niederschlag saugte man ab, wusch mit Ether nach und erhielt 1,5 g kristallinen 7-<(2-(2-Tritylaminothiazol-4-yl)-2-(Z)-1-methyl-1-methoxyethoximinoacetamido)>-3-methoxymethyl-3-cephem-4-carbonsäure-1-(1-methoxyprop-2-yloxycarbonyloxy)-ethylester. Aus dem Filtrat erhielt man durch Behandlung mit Pentan nochmals 0,34 g, zusammen 1,84 g (41 %), der Titelverbindung.

$^1$H-NMR ($d_6$-DMSO): $\delta$ = (ppm)
9.54 (2 x d, CONH, J = 8 Hz)
8.85 (s, NH-Trityl)
7.4-7.2 (m, Phenyl-H)
6.85-6.8 (2 x g, O-CH-O)
6.7 (2 x s, Thiazol-H)
5.75 (2 x q, J = 5 Hz, C-7-H)
5.2 (2 x d, C-6-H)
4.85 (m, $OCO_2$-CH)
4.15 (m, -$CH^2$-O)
3.55 (m, C-2-H)
3.4 (q, verdeckt, J = 5 Hz, $CH_2$-$OCH_3$)
3,25 (s, $CH_2$-$OCH_3$)
3.2 (s, 3-$CH_2$-$OCH_3$)
3.1 (s, O-C-$O_2CH_3$)
1.5 (d, J = 6 Hz, $CO_2$-CH($CH_3$)-$OCO_2$)
1.4 (s, C-$(CH_3)_2$)
1.2 (3 x t, J = 7 Hz, $CH_3$-CH-$OCO_2$).

Analog Beispiel 1, Schritt A und B, wurden die in der Tabelle 2 zusammengestellten Verbindungen als amorphe Feststoffe erhalten.

Tabelle 2

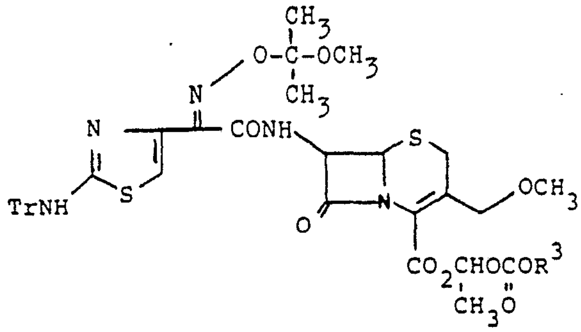

| Beisp. Nr. | $R^3$ | Ausbeute | NMR ($d_6$-DMSO) δ = (ppm) |
|---|---|---|---|
| 1 | 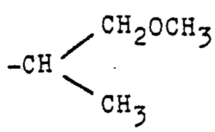 | 41 % | 9.54 (2 x d, CONH, J=8 Hz)<br>8.85 (s, NH-Trityl)<br>7.4-7.2 (m, Phenyl-H)<br>6.85-6.8 (2 x q, O-CH-O)<br>6.7 (2 x s, Thiazol-H)<br>5.75 (2 x q, J=5 Hz, C-7-H)<br>5.2 (2 x d, C-6-H)<br>4.85 (m, $OCO_2$-CH)<br>4.15 (m, 3-$CH_2$-O)<br>3.55 (m, C-2-H)<br>3.4 (q, verdeckt, J=5 Hz, $CH_2$-$OCH_3$)<br>3.25 (s, $CH_2$-$OCH_3$)<br>3.2 (s, 3-$CH_2$-$OCH_3$)<br>3.1 (s, O-C-$OCH_3$)<br>1.5 (d, J=6 Hz, $CO_2$-CH($CH_3$)-$OCO_2$)<br>1.4 (s, C-$(CH_3)_2$)<br>1.2 (3 x t, J=7 Hz, $CH_3$-CH-$OCO_2$). |

Tabelle 2 (Fortsetzung)

| Beisp. Nr. | $R^3$ | Ausbeute | NMR ($d_6$-DMSO) δ = (ppm) |
|---|---|---|---|
| 2 | 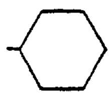 | 39 % | 9.52 (2 x d, CONH, J=8 Hz)<br>8.85 (s, NH-Trityl)<br>7.4-7.2 (m, Phenyl-H)<br>6.85-6.75 (2 x q, O-CH-O)<br>6.7 (2 x s, Thiazol-H)<br>5.75 (2 x q, J=5 Hz, C-7-H)<br>5.15 (2 x d, C-6-H)<br>4.55 (m, $OCO_2$-CH)<br>4.15 (m, 3-$CH_2$-O)<br>3.55 (2 x AB, C-2-H)<br>3.2 (s, 3-$CH_2$-$OCH_3$)<br>3.1 (s, O-C-$OCH_3$)<br>1.85-1.25 (m, Cyclohexyl-H)<br>1.5 (d, J=6 Hz, $CO_2$-CH($CH_3$)-$OCO_2$, verdeckt)<br>1.4 (s, C-($CH_3$)$_2$, verdeckt) |
| 3 | 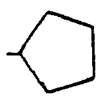 | 38 % | 9.55 (2 x d, CONH, J=8 Hz)<br>8.85 (s, NH-Trityl)<br>7.4-7.2 (m, Phenyl-H)<br>6.8-6.75 (2 x q, O-CH-O)<br>6.7 (2 x s, Thiazol-H)<br>5.75 (2 x q, J=5 Hz, C-7-H)<br>5.17 (2 x d, C-6-H)<br>5.05 (m, $OCO_2$-CH)<br>4.15 (m, 3-$CH_2$-O)<br>3.55 (2 x AB, C-2-H)<br>3.2 (s, 3-$CH_2$-$OCH_3$)<br>3.1 (s, O-C-$OCH_3$)<br>1.85-1.25 (m, Cyclopentyl-H)<br>1.45 (2 x d, J=6 Hz, $CO_2$-CH($CH_3$)-$OCO_2$, verdeckt)<br>1.4 (s, C-($CH_3$)$_2$, verdeckt) |

Tabelle 2 (Fortsetzung)

| Beisp. Nr. | $R^3$ | Ausbeute | NMR ($d_6$-DMSO) δ = (ppm) |
|---|---|---|---|
| 4 | 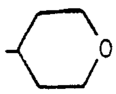 | 32 % | 9.5 (2 x d, CONH, J=8 Hz)<br>8.85 (s, NH-Trityl)<br>7.4-7.2 (m, Phenyl-H)<br>6.92-6.77 (2 x q, O-CH-O)<br>6.69 (2 x s, Thiazol-H)<br>5.75 (2 x q, J=5 Hz, C-7-H)<br>5.18 (2 x d, C-6-H)<br>4.77 (m, $OCO_2$-CH)<br>4.15 (m, 3-$CH_2$-O)<br>3.75 (m, $(CH_2)_2O$)<br>3.55 (2 x AB, C-2-H)<br>3.2 (s, 3-$CH_2$-$OCH_3$)<br>3.1 (s, O-C-$OCH_3$)<br>1.59 (m, $CH(CH_2)_2$)<br>1.5 (2 x d, J=6 Hz, $CO_2$-$CH(CH_3)$-$OCO_2$)<br>1.4 (s, C-$(CH_3)_3$) |

**Beispiel 5**

7-<(2-(2-Aminothiazol-4-yl)-2-(Z)-hydroxyiminoacetamido)-3-methoxymethyl-3-cephem-4-carbonsäure-1-(1-methoxyprop-2-yloxycarbonyloxy)-ethylester

1,83 g (2 mmol) des nach Beispiel 1 erhaltenen Cephalosporins wurde bei Raumtemperatur in 18 ml 90 %iger Ameisensäure gelöst. Die Lösung verdünnte man mit 2 ml Wasser, rührte 40 Minuten bei Raumtemperatur und saugte das ausgefallene Triphenylmethanol ab. Das Filtrat engte man unter Zusatz von Toluol ein, nahm den öligen Rückstand in Aceton auf und klärte die Lösung unter Zusatz von Aktivkohle. Das klare Filtrat versetzte man unter Eiskühlung mit n-Pentan, wobei sich das Produkt als Öl abschied. Man dekantierte das Lösungsmittel ab und verrieb den öligen Rückstand mit n-Pentan, wobei das amorphe Produkt ausfiel. Ausbeute: 0,74 g (64 %).

$^1$H-NMR ($d_6$-DMSO): δ = (ppm)
11.4 (s, NOH)
9.5 (d, J = 8 Hz, CONH)
7.3 (s, $NH_2$
6.85-6.8 (2 x q, J = 6 Hz, O-CH-O)
6.7 (s, Thiazol-H)
5.85 (2 x q, J = 5 Hz, C-7-H)
5.2 (3 x d, C-6-H)
4.85 (m, CH-$OCO_2$)
4.15 (s, 3-$CH_2$-O)
3.55 (A/B, C-2-H)
3.25 (s, 3-$CH_2$-$OCH_3$)
3.2 (s, $CH_2$-$OCH_3$)
1.5 (2 x d, J = 6 Hz, $CO_2$-$CH(CH_3)$-$OCO_2$)
1.2 (3 x 7, $CH_3$-CH-$OCO_2$).

Analog Beispiel 5 wurden die in der Tabelle 3 aufgeführten Verbindungen als amorphe Feststoffe erhalten.

Tabelle 3

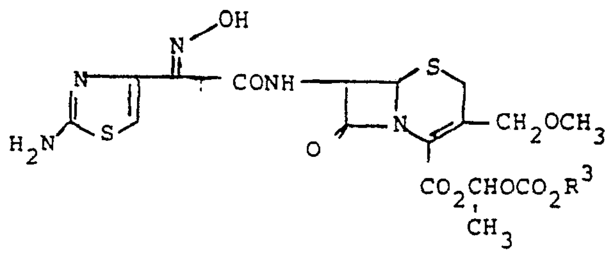

| Beisp. Nr. | $R^3$ | Ausbeute | NMR ($d_6$-DMSO) δ = (ppm) |
|---|---|---|---|
| 5 | 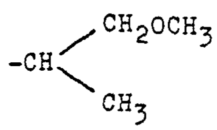 | 64 % | 11.4 (s, NOH)<br>9.5 (d, J=8 Hz, CONH)<br>7.3 (s, $NH_2$)<br>6.85 - 6.8 (2 x q, J=6 Hz, O-CH-O)<br>6.7 (s, Thiazol-H)<br>5.85 (2 x q, J=5 Hz, C-7-H)<br>5.2 (3 x d, C-6-H)<br>4.85 (m, $CH-OCO_2$)<br>4.15 (s, $3-CH_2-O$)<br>3.55 (A/B, C-2-H)<br>3.25 (s, $3-CH_2-OCH_3$)<br>3.2 (s, $CH_2-OCH_3$)<br>1.5 (2 x d, J=6 Hz, $CO_2-CH(CH_3)-OCO_2$<br>1.2 (3 x t, $CH_3-CH-OCO_2$). |
| 6 | 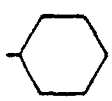 | 69 % | 11.3 (s, NOH)<br>9.45 (d, J=8 Hz, CONH)<br>7.1 (s, $NH_2$)<br>6.85-6.8 (2 x q, J=6 Hz, O-CH-O)<br>6.65 (s, Thiazol-H)<br>5.83 (2 x q, J=5 Hz, C-7-H)<br>5.15 (2 x d, C-6-H)<br>4.59 (m, $CH-OCO_2$)<br>4.15 (s, $3-CH_2-O$)<br>3.55 (2 x AB, C-2-H)<br>3.2 (s, $3-CH_2-OCH_3$)<br>1,85-1.25 (m, Cyclohexyl-H)<br>1.5 (2 x d, J=6 Hz, $CO_2-CH(CH_3)-OCO_2$, verdeckt). |

Tabelle 3

| Beisp. Nr. | $R^3$ | Ausbeute | NMR ($d_6$-DMSO) $\delta$ = (ppm) |
|---|---|---|---|
| 7 | 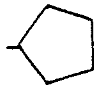 | 58 % | 11.3 (s, NOH)<br>9.45 (d, J=8 Hz, CONH)<br>7.15 (s, $NH_2$)<br>6.85-6.75 (2 x q, J=6 Hz, O-CH-O)<br>6.65 (s, Thiazol-H)<br>5.83 (2 x q, J=5 Hz, C-7-H)<br>5.2 (2 x d, C-6-H)<br>5.05 (m, CH-$OCO_2$)<br>4.15 (s, 3-$CH_2$-O)<br>3.55 (2 x AB, C-2-H)<br>3.2 (s, 3-$CH_2$-$OCH_3$)<br>1.85-1.5 (m, Cyclopentyl-H)<br>1.5 (2 x d, J=6 Hz, $CO_2$-CH($CH_3$)-$OCO_2$, verdeckt) |
| 8 | 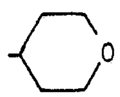 | 55 % | 11.3 (s, NOH)<br>9.45 (d, J=8 Hz, CONH)<br>7.12 (s, $NH_2$)<br>6.91-6.75 (2 x q, J=6 Hz, O-CH-O)<br>6.65 (s, Thiazol-H)<br>5.85 (2 x q, J=5 Hz, C-7-H)<br>5.19 (2 x d, C-6-H)<br>4.79 (m, CH-$OCO_2$)<br>4.15 (s, 3-$CH_2$-O)<br>3.78 (m, $(CH_2)_2O$)<br>3.55 (2 x AB, C-2-H)<br>3.2 (s, 3-$CH_2$-$OCH_3$)<br>1.6 (m, $C(CH_2)_2$)<br>1.5 (2 x d, J=6 Hz, $CO_2$-CH($CH_3$)-$OCO_2$, verdeckt) |

**Beispiel 9**

7-<(2-(2-Aminothiazol-4-yl)-2-(Z)-methoxyiminoacetamido>-3-methyl-3-cephem-4-carbonsäure-1-(isopropoxycarbonyloxy)ethylester

Schritt A

Zu einer Suspension von 200 mg (1.5 mmol) wasserfreiem Zinkchlorid in 10 ml Schwefelkohlenstoff gab man nacheinander 1,5 g (10 mmol) Natriumjodid und 1,2 g (7.5 mmol) 1-Chlorethylisopropyl-carbonat. Man rührte 2 Stunden unter Stickstoffatmosphäre, goß man die Reaktionsmischung in ein Gemisch aus 9 %iger $NaHCO_3$-Lösung und Ether und trennte die Phasen. Die organische Phase wurde gewaschen ($NaHCO_3$-, Natriumthiosulfat- und NaCl- Lösung) und getrocknet ($MgSO_4$). Das Lösungsmittel destillierte man im Vakuum bei 20°C ab und erhielt rohes 1-Jodethyl-isopropyl-carbonat als farbloses Öl, das ohne Reinigung sofort weiterverwendet wurde (Schritt B).

Schritt B

Das aus Schritt A erhaltene Rohprodukt wurde in 18 ml trockenem DMF aufgenommen und unter Eiskühlung zu einer Lösung von 1,3 g (3 mmol) Kaliumcefetamet in 10 ml trockenem DMF gegeben. Nach 75 Minuten rührte man das Reaktionsgemisch in eine Mischung von 9 %iger $NaHCO_3$-Lösung und Essigester ein. Die organische Phase wurde gewaschen ($NaHCO_3$- und NaCl- Lösung), getrocknet ($MgSO_4$) und das Lösungsmittel im Vakuum abgezogen. Das Rohprodukt chromatographierte man an 100 g Kieselgel mit Ethylacetat. Man erhielt 300 mg der Titelverbindung.

$^1$H-MNR ($d_6$-DMSO): $\delta$ = (ppm)
9.6 (2 x d, CONH, J = 8Hz)
7.23 (br, s, $NH_2$)
6.78 und 6.83 (2 x q, O-CH-O)
6.73 (2 x s, Thiazol-H)
5.77 (2 x q, J = 5 Hz, C-7-H)
5.13 (2 x d, C-6-H)
3.85 (s, $OCH_3$)
3.4-3.68 (m, C-2-H)
2.03 (s, $CH_3$)
1.5 (d, J = 6 Hz, $Co_2$-CH($CH_3$)-$OCO_2$)
1.25 (d, $(CH_3)_2$-CH).

Analog Beispiel 9 wurden die in Tabelle 4 aufgeführten Verbindungen als amorphe Feststoffe erhalten.

Tabelle 4

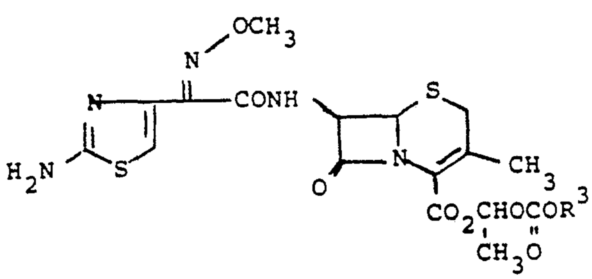

| Beisp. Nr. | $R^3$ | Ausbeute | NMR ($d_6$-DMSO) $\delta$ = (ppm) |
|---|---|---|---|
| 9 | 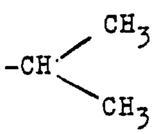 | 47 % | 9.6 (2 x d, CONH, J=8 Hz)<br>7.23 (br s, $NH_2$)<br>6.78 und 6.83 (2 x q, O-CH-O)<br>6.73 (2 x s, Thiazol-H)<br>5.77 (2 x q, J=5 Hz, C-7-H)<br>5.13 (2 x d, C-6-H)<br>3.85 (s, $OCH_3$)<br>3.4 - 3.68 (m, C-2-H)<br>2.03 (s, $CH_3$)<br>1.5 (d, J=6 Hz, $CO_2$-CH($CH_3$)-$OCO_2$)<br>1.25 (d, $(CH_3)_2$-CH). |

Tabelle 4 (Fortsetzung)

| Beisp. Nr. | $R^3$ | Ausbeute | NMR ($d_6$-DMSO) δ = (ppm) |
|---|---|---|---|
| 10 | 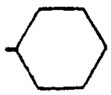 | 42 % | 9.6 (2 x d, CONH, J=8 Hz)<br>7.23 (br s, $NH_2$)<br>6.78 und 6.83 (2 x q, O-CH-O)<br>6.73 (2 x s, Thiazol-H)<br>5.77 (2 x q, J=5 Hz, C-7-H)<br>5.13 (2 x d, C-6-H)<br>4.58 und 4.4 (m, 1H, O-CH)<br>3.85 (s, $OCH_3$)<br>3.38 - 3.68 (m, C-2-H)<br>2.03 (s, $CH_3$)<br>1.5 (d, J=6 Hz, $CO_2$-CH($CH_3$)-$OCO_2$)<br>1.05 - 1.9 (m, 10H, Cyclohexyl-H). |
| 11 | 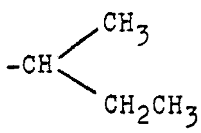 | 46 % | 9.57 (2 x d, CONH, J=8 Hz)<br>7.20 (br s, $NH_2$)<br>6.78 und 6.83 (2 x q, O-CH-O)<br>6.73 (2 x s, Thiazol-H)<br>5.73 (2 x q, J=5 Hz, C-7-H)<br>5.13 (2 x d, C-6-H)<br>4.62 (m, 1H, O-CH)<br>3.82 (s, $OCH_3$)<br>3.38 - 3.68 (m, C-2-H)<br>2.03 (s, $CH_3$)<br>1.5 (d, J=6 Hz, $CO_2$-CH($CH_3$)-$OCO_2$)<br>1.58, 1.2, 0.85 (m, 8H, $-CH_2-$ und $-CH_3$) |

Tabelle 4 (Fortsetzung)

| Beisp. Nr. | $R^3$ | Ausbeute | NMR ($d_6$-DMSO) δ = (ppm) |
|---|---|---|---|
| 12 | $-\underset{CH_3}{CH}CH_2OCH_3$ | 43 % | 9.6 (2 x d, CONH, J=8 Hz)<br>7.23 (br s, $NH_2$)<br>6.78 und 6.83 (2 x q, O-CH-O)<br>6.73 (2 x s, Thiazol-H)<br>5.77 (2 x q, J=5 Hz, C-7-H)<br>5.15 (2 x d, C-6-H)<br>4.82 (m, 1H, O-CH)<br>3.85 (s, N-$OCH_3$)<br>3.4 - 3.68 (m, C-2-H)<br>3.4 (d, $CH_2$)<br>3.25 (s, 3H, $OCH_3$)<br>2.03 (s, $CH_3$)<br>1.5 (d, J=6 Hz, $CO_2$-CH($CH_3$)-$OCO_2$)<br>1.2 (m, 3H, $CH_3$) |
| 13 | $-CH_2CH_2CH_3$ | 47 % | 7.23 (br s, $NH_2$)<br>6.78 und 6.83 (2 x q, O-CH-O)<br>6.73 (2 x s, Thiazol-H)<br>5.7 - 5.8 (2 x q, J=5 Hz, C-7-H)<br>5.13 (2 x d, C-6-H)<br>4.09 (2 x t, 2H, O-$CH_2$)<br>3.85 (s, $OCH_3$)<br>3.38 - 3.68 (m, C-2-H)<br>2.03 (s, $CH_3$)<br>1.62 (m, 2H, -$CH_2$-)<br>1.5 (d, J=6 Hz, $CO_2$-CH($CH_3$)-$OCO_2$)<br>0.89 (t, -$CH_3$). |
| 14 | $-CH_2CH_2OCH_3$ | 39 % | 9.59 (2 x d, CONH, J=8 Hz)<br>7.23 (br s, $NH_2$)<br>6.78 und 6.83 (2 x q, O-CH-O)<br>6.73 (2 x s, Thiazol-H)<br>5.77 (2 x q, J=5 Hz, C-7-H)<br>5.13 (2 x d, C-6-H)<br>4.25 (m, 2H, O-$CH_2$)<br>3.85 (s, N-$OCH_3$)<br>3.4 - 3.68 (m, C-2-H)<br>3.52 (m, 2H, O-$CH_2$)<br>3.25 (s, 3H, $OCH_3$)<br>2.03 (s, $CH_3$)<br>1.5 (d, J=6 Hz, $CO_2$-CH($CH_3$)-$OCO_2$) |

**Tabelle 4** (Fortsetzung)

| Beisp. Nr. | $R^3$ | Ausbeute | NMR ($d_6$-DMSO) δ = (ppm) |
|---|---|---|---|
| 15 | 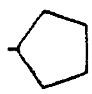 | 42 % | 9.6 (2 x d, CONH, J=8 Hz)<br>7.23 (br s, $NH_2$)<br>6.78 und 6.83 (2 x q, O-CH-O)<br>6.73 (2 x s, Thiazol-H)<br>5.77 (2 x q, J=5 Hz, C-7-H)<br>5.13 (2 x d, C-6-H)<br>3.85 (s, $OCH_3$)<br>3.4 - 3.68 (m, C-2-H)<br>2.03 (s, $CH_3$)<br>1.5 (d, J=6 Hz, $CO_2$-CH($CH_3$)-$OCO_2$) |
| 16 | 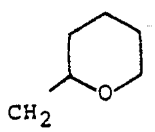 | 31 % | 9.6 (2 x d, CONH, J=8 Hz)<br>7.23 (br s, $NH_2$)<br>6.78 und 6.83 (2 x q, O-CH-O)<br>6.73 (2 x s, Thiazol-H)<br>5.77 (2 x q, J=5 Hz, C-7-H)<br>5.13 (2 x d, C-6-H)<br>4.09 (m, 3H, O-$CH_2$ und 2'-H)<br>3.85 (s, $OCH_3$)<br>3.4 - 3.68 (m, C-2-H)<br>2.03 (s, $CH_3$)<br>1.8 (m)<br>1.5 (d, J=6 Hz, $CO_2$-CH($CH_3$)-$OCO_2$) |
| 17 | 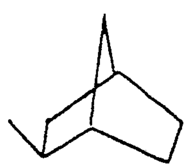 | 38 % | 9.6 (2 x d, CONH, J=8 Hz)<br>7.22 (br s, $NH_2$)<br>6.78 und 6.82 (2 x q, O-CH-O)<br>6.73 (2 x s, Thiazol-H)<br>5.75 (2 x q, J=5 Hz, C-7-H)<br>5.13 (2 x d, C-6-H)<br>4.5 (m, 1H, 2'-H)<br>3.85 (s, $OCH_3$)<br>3.4 - 3.68 (m, C-2-H)<br>2.03 (s, $CH_3$)<br>1.5 (d, J=6 Hz, $CO_2$-CH($CH_3$)-$OCO_2$) |

Tabelle 4 (Fortsetzung)

| Beisp. Nr. | $R^3$ | Ausbeute | NMR ($d_6$-DMSO) δ = (ppm) |
|---|---|---|---|
| 18 | 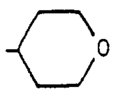 | 35 % | 9.6 (2 x d, CONH, J=8 Hz)<br>7.21 (br s, $NH_2$)<br>6.79 und 6.88 (2 x q, O-CH-O)<br>6.73 (2 x s, Thiazol-H)<br>5.76 (2 x q, J=5 Hz, C-7-H)<br>5.13 (2 x d, C-6-H)<br>4.8 (m, 1H, 4'-H)<br>3.85 (s, $OCH_3$)<br>3.4 - 3.68 (m, C-2-H)<br>2.03 (s, $CH_3$)<br>1.5 (d, J=6 Hz, $CO_2-CH(CH_3)-OCO_2$) |
| 19 | $-CH-CH_2CH_3$<br>$CH_3$ (R) | 45 % | 9.55 (2 x d, CONH, J=8 Hz)<br>7.20 (br s, $NH_2$)<br>6.78 und 6.83 (2 x q, O-CH-O)<br>6.73 (2 x s, Thiazol-H)<br>5.74 (2 x q, J=5 Hz, C-7-H)<br>5.13 (2 x d, C-6-H)<br>4.63 (q, 2H, $CH_2-CH_3$)<br>3.85 (s, $OCH_3$)<br>3.4 - 3.68 (m, C-2-H)<br>2.03 (s, $CH_3$)<br>1.51 (d, J=6 Hz, $CO_2-CH(CH_3)-OCO_2$)<br>1.2 (2 x t, 3H, $O-CH(CH_3)-CH_2-$)<br>0.83 (t, 3H, $CH_3$) |
| 20 | $-CH_2CH_2CH_3$<br>$CH_3$ (S) | 45 % | 9.55 (2 x d, CONH, J=8 Hz)<br>7.20 (br s, $NH_2$)<br>6.78 und 6.83 (2 x q, O-CH-O)<br>6.73 (2 x s, Thiazol-H)<br>5.74 (2 x q, J=5 Hz, C-7-H)<br>5.13 (2 x d, C-6-H)<br>4.63 (2 x q, 2H, $CH_2-CH_3$)<br>3.85 (s, $OCH_3$)<br>3.4 - 3.68 (m, C-2-H)<br>2.03 (s, $CH_3$)<br>1.51 (d, J=6 Hz, $CO_2-CH(CH_3)-OCO_2$)<br>1.2 (2 x t, 3H, $O-CH(CH_3)-CH_2-$)<br>0.83 (2 x t, 3H, $CH_3$) |

Tabelle 4 (Fortsetzung)

| Beisp. Nr. | $R^3$ | Ausbeute | NMR ($d_6$-DMSO) δ = (ppm) |
|---|---|---|---|
| 21 | $-CH_2CH_2CH_2OCH_2CH_3$ | 38 % | 9.57 (2xd, CONH, J = 8 Hz)<br>7.20 (br s, $NH_2$)<br>6.78 und 6.83 (2 x q, O-CH-O)<br>6.73 (2 x s, Thiazol-H)<br>5.74 (2 x q, J=5 Hz, C-7-H)<br>5.13 (2 x d, C-6-H)<br>4.19 (m, 2H, $CO_2CH_2$)<br>3.85 (s, $OCH_3$)<br>3.4 - 3.68 (m, C-2-H)<br>3.4 (m, 2H, $O-CH_2CH_3$)<br>2.03 (s, $CH_3$)<br>1.84 (m, 2H, $CH_2CH_2CH_2$)<br>1.5 (d, J=6 Hz, $CO_2-CH(CH_3)-OCO_2$<br>1.1 (t, 3H, $CH_3$) |
| 22 | 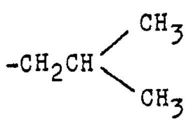 | 40 % | 9.57 (2 x d, CONH, J=8 Hz)<br>7.21 (br s, $NH_2$)<br>6.78 und 6.83 (2 x q, O-CH-O)<br>6.73 (2 x s, Thiazol-H)<br>5.74 (2 x q, J=5 Hz, C-7-H)<br>5.13 (2 x d, C-6-H)<br>3.92 (2 x d, 2H, $OCH_2CH$)<br>3.85 (s, $OCH_3$)<br>3.4 - 3.68 (m, C-2-H)<br>2.03 (s, $CH_3$)<br>1.9 (m, 1H, $-CH(CH_3)_2$)<br>1.5 (d, J=6 Hz, $CO_2-CH(CH_3)-OCO_2$<br>0.89 (2 x d, $CH(CH_3)_2$) |

## Ansprüche

1. Cephemcarbonsäureester der allgemeinen Formel

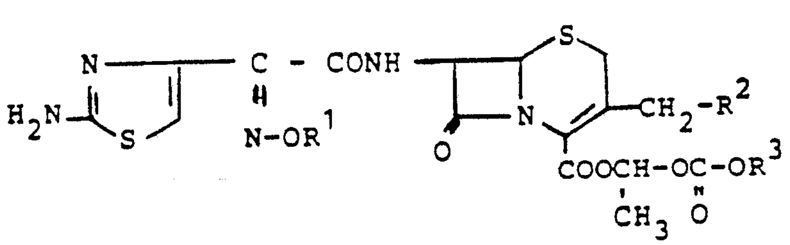

worin bedeuten

$R^1$ Wasserstoff oder Methyl und

$R^2$ Wasserstoff ode Methoxy, wobei immer einer der beiden Substituenten $R^1$ oder $R^2$ für Wasserstoff steht;

$R^3$ geradkettiges oder verzweigtes $C_1$-$C_5$-Alkyl, das substituiert sein kann durch $C_1$-$C_3$-Alkoxy, $C_3$-$C_8$-Cycloalkyl oder $C_2$-$C_7$-Cycloalkoxy; $C_3$-$C_8$-Cycloalkyl oder $C_2$-$C_7$-Cycloalkoxy,

wobei für den Fall, daß $R^1$ Wasserstoff und $R^2$ Methoxy ist, $R^3$ nicht $C_1$-$C_4$-Alkyl sein kann und worin die Gruppe -$OR^1$ in syn-Position steht, sowie deren physiologisch verträgliche Säureadditionssalze.

2. Verfahren zur Herstellung von Cephemcarbonsäureestern der allgemeinen Formel I

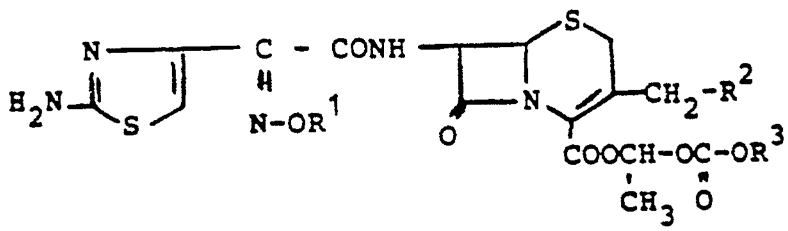 (I)

worin bedeuten

$R^1$ Wasserstoff oder Methyl und

$R^2$ Wasserstoff oder Methoxy, wobei immer einer der beiden Substituenten $R^1$ oder $R^2$ für Wasserstoff steht,

$R^3$ geradliniges oder verzweigtes $C_1$-$C_5$-Alkyl, das substituiert sein kann durch $C_1$-$C_3$-Alkoxy, $C_3$-$C_8$-Cycloalkyl oder $C_2$-$C_7$-Cycloalkoxy, $C_3$-$C_8$-Cycloalkyl oder $C_2$-$C_7$-Cycloalkoxy,

wobei für den Fall, daß $R^1$ Wasserstoff und $R^2$ Methoxy ist, $R^3$ nicht $C_1$-$C_4$-Alkyl sein kann und worin die Gruppe -$OR^1$ in syn-Position steht, sowie von deren physiologisch verträglichen Säureadditionssalzen, dadurch gekennzeichnet, daß man

a) eine Verbindung der Formel II

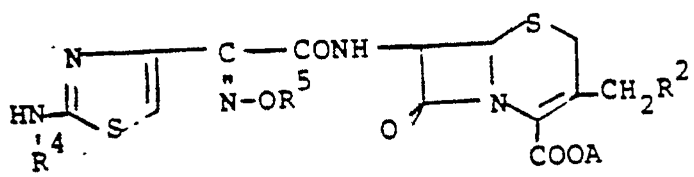 (II)

in der $R^2$ für Wasserstoff oder Methoxy, $R^4$ für Wasserstoff oder eine Aminoschutzgruppe, $R^5$ für Methyl oder eine leicht abspaltbare Gruppe und A ein Kation steht, wobei $R^4$ nur dann für Wasserstoff stehen kann, wenn $R^5$ Methyl ist,

mit einer Verbindung der allgemeinen Formel III

$$X - \underset{CH_3}{CH} - OCO_2 - R^3 \qquad (III)$$

worin $R^3$ die obige Bedeutung besitzt und X für eine Abgangsgruppe steht, zu dem Ester der allgemeinen Formel IV umsetzt

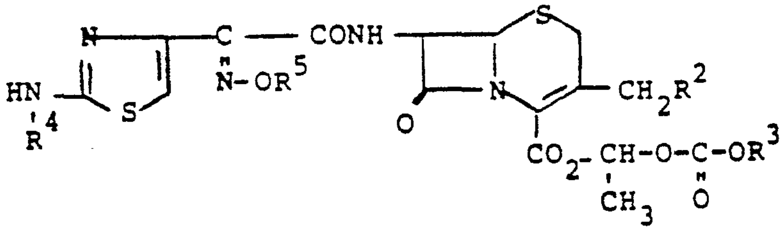 (IV)

und die Gruppen $R^4$ und $R^5$ in der Bedeutung einer Schutzgruppe oder leicht abspaltbaren Gruppe in an sich bekannter Weise entfernt oder

b) eine Verbindung der allgemeinen Formel V

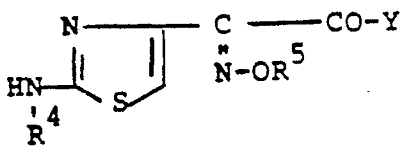

(V)

in der $R^4$ und $R^5$ die vorstehende Bedeutung besitzen und Y für eine aktivierende Gruppe steht, mit einer Verbindung der allgemeinen Formel VI

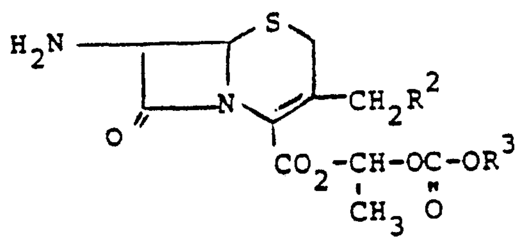

(VI)

in der $R^2$ und $R^3$ die vorstehende Bedeutung besitzen oder mit einem Salz dieser Verbindung, zu einer Verbindung der allgemeinen Formel IV umsetzt und die Gruppen $R^4$ und $R^5$ in der Bedeutung einer Schutzgruppe oder einer leicht abspaltbaren Gruppe in an sich bekannter Weise abspaltet, oder

c) eine Verbindung der allgemeinen Formel VII

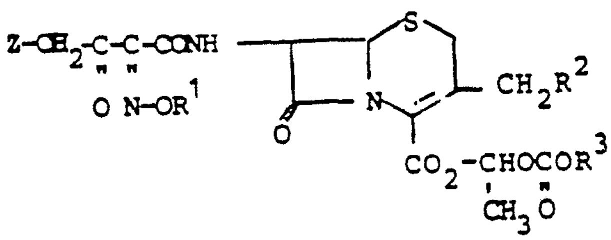

(VII)

in der Z für Halogen steht und $R^1$, $R^2$ und $R^3$ die vorstehende Bedeutung besitzen, mit Thioharnstoff zu Verbindungen der allgemeinen Formel I umsetzt und -falls erwünscht - die erhaltenen Verbindungen in ein physiologisch verträgliches Säureadditionssalz überführt.

3. Gegen bakterielle Infektionen wirksame pharmazeutische Zubereitungen gekennzeichnet durch einen Gehalt an Cephemcarbonsäureestern der allgemeinen Formel I.

4. Verfahren zur Herstellung von gegen bakterielle Infektionen wirksamen pharmazeutischen Zubereitungen, dadurch gekennzeichnet, daß ein Cephemcarbonsäureester der allgemeinen Formel I mit pharmazeutisch üblichen Trägerstoffen oder Verdünnungsmitteln in eine pharmazeutisch geeignete Verabreichungsform gebracht wird.

5. Verwendung von Cephemcarbonsäureestern der allgemeinen Formel I zur Bekämpfung bakterieller Infektionen.

Patentansprüche für folgende Vertragsstaaten: ES, GR

1. Verfahren zur Herstellung von Cephemcarbonsäureestern der allgemeinen Formel I

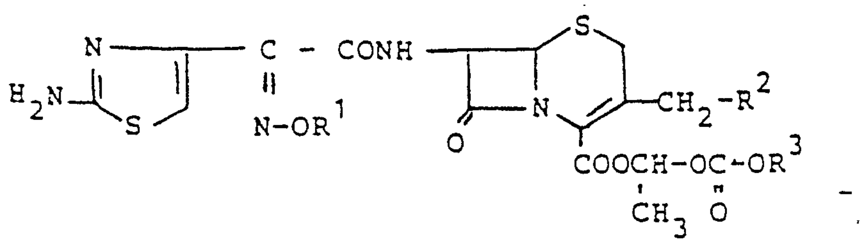

(I)

worin bedeuten

$R^1$ Wasserstoff oder Methyl und

$R^2$ Wasserstoff oder Methoxy, wobei immer einer der beiden Substituenten $R^1$ oder $R^2$ für Wasserstoff steht,

$R^3$ geradliniges oder verzweigtes $C_1$-$C_5$-Alkyl, das substituiert sein kann durch $C_1$-$C_3$-Alkoxy, $C_3$-$C_8$-Cycloalkyl oder $C_2$-$C_7$-Cycloalkoxy, $C_3$-$C_8$-Cycloalkyl oder $C_2$-$C_7$-Cycloalkoxy,

wobei für den Fall, daß $R^1$ Wasserstoff und $R^2$ Methoxy ist, $R^3$ nicht $C_1$-$C_4$-Alkyl sein kann und worin die Gruppe -$OR^1$ in syn-Position steht, sowie von deren physiologisch verträglichen Säureadditionssalzen, dadurch gekennzeichnet, daß man

a) eine Verbindung der Formel II

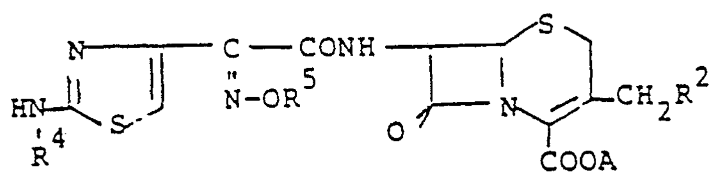

(II)

in der $R^2$ für Wasserstoff oder Methoxy, $R^4$ für Wasserstoff oder eine Aminoschutzgruppe, $R^5$ für Methyl oder eine leicht abspaltbare Gruppe und A ein Kation steht, wobei $R^4$ nur dann für Wasserstoff stehen kann, wenn $R^5$ Methyl ist,

mit einer Verbindung der allgemeinen Formel III

$$X - \underset{\displaystyle CH_3}{\underset{|}{CH}} - OCO_2 - R^3 \qquad (III)$$

worin $R^3$ die obige Bedeutung besitzt und X für eine Abgangsgruppe steht, zu dem Ester der allgemeinen Formel IV umsetzt

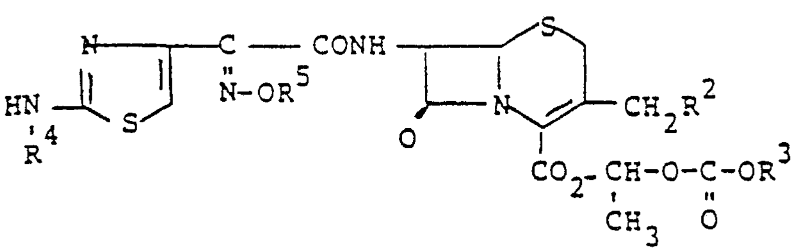

(IV)

und die Gruppen $R^4$ und $R^5$ in der Bedeutung einer Schutzgruppe oder leicht abspaltbaren Gruppe in an sich bekannter Weise entfernt oder

b) eine Verbindung der allgemeinen Formel V

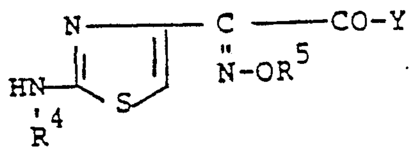

(V)

in der $R^4$ und $R^5$ die vorstehende Bedeutung besitzen und Y für eine aktivierende Gruppe steht, mit einer Verbindung der allgemeinen Formel VI

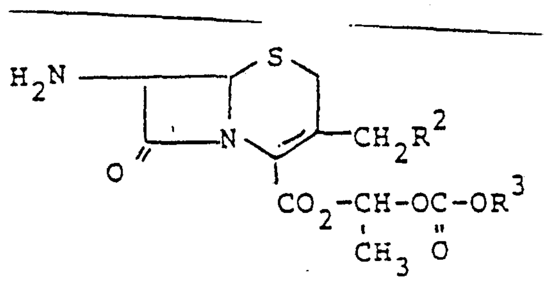

(VI)

in der $R^2$ und $R^3$ die vorstehende Bedeutung besitzen oder mit einem Salz dieser Verbindung, zu einer Verbindung der allgemeinen Formel IV umsetzt und die Gruppen $R^4$ und $R^5$ in der Bedeutung einer Schutzgruppe oder einer leicht abspaltbaren Gruppe in an sich bekannter Weise abspaltet, oder

c) eine Verbindung der allgemeinen Formel VII

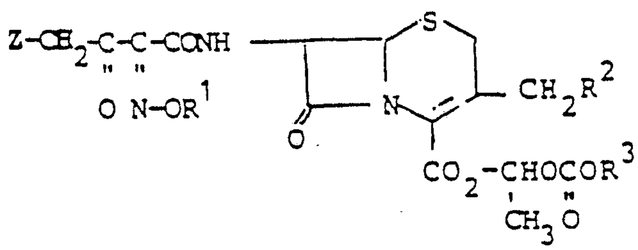

(VII)

in der Z für Halogen steht und $R^1$, $R^2$ und $R^3$ die vorstehende Bedeutung besitzen, mit Thioharnstoff zu Verbindungen der allgemeinen Formel I umsetzt und -falls erwünscht - die erhaltenen Verbindungen in ein physiologisch verträgliches Säureadditionssalz überführt.

2. Verwendung von Cephemcarbonsäureestern der allgemeinen Formel I zur Herstellung von Arzneimitteln zur Bekämpfung bakterieller Infektionen.